# EUROPEAN PATENT SPECIFICATION

(11) **EP 0 418 187 B1**
(45) Date of publication and mention of the grant of the patent: **14.12.1994**
(21) Application number: 90610050.8
(22) Date of filing: 02.08.1990
(51) Int. Cl.: C12M 1/02

(54) **Method and apparatus for performing a fermentation**
Verfahren und Vorrichtung zur Durchführung einer Fermentation
Procédé et appareil pour effectuer une fermentation

(30) Priority: 07.08.1989 DK 3867/89
(43) Date of publication of application: 20.03.1991
(73) Proprietor: DANSK BIOPROTEIN A/S, DK-5230 Odense M (DK)
(72) Inventor: Jorgensen, Lars, DK-5230 Odense M (DK)
(74) Representative: Larsen, Hans Ole

(56) References cited:
- EP-A- 0 027 911
- EP-A- 0 185 407
- FR-A- 849 162
- FR-A- 2 229 451
- FR-A- 2 264 869

## Description

The present invention relates to a method for performing a fermentation in which method an introduction and a subsequent mixing of one or more gases is carried out in a fermentation fluid, which is found in an apparatus of the loop-reactor type, which apparatus has a top part and a U-part consisting of a downstream part, a U-bend part and an upstream part, the fermentation fluid being circulated in the apparatus by means of a pump placed in the U-part.

The present invention also relates to an apparatus for use in this method, which apparatus is of the loop reactor type and has a top part and a U-part consisting of a substantially vertical downstream part, a U-bend part and a substantially vertical upstream part, a pump placed in the U-part, which pump is adapted to circulate a fermentation fluid in the apparatus, and devices for introduction of one or more gases into the fermentation fluid, whereby the top part is made up of a cylinder having a substantially larger diameter than the downstream part and being connected hereto via a taper part.

The method of the invention and the apparatus of the invention may be used when performing the type of fermentations in which one or more gases are introduced into the fermentation fluid and in which microorganisms and/or other fermentation products are formed.

In the traditional bioreactors (fermentors) the mixing of the gases in the fermentation fluid takes place by means of agitator blades placed centrally in the fermentor. The agitator blades cause turbulence in the fluid which means that gas - as a rule injected at the bottom of the reactor - is being spread within the fluid in the form of small gas bubbles from which the gas molecules diffuse into the fluid. This type of reactor gives a relatively homogeneous mixing or dispersion, i. e. that if a measurement is made at the top or at the bottom of the reactor approximately the same concentration of gases and other components will be found. This type of reactor is not particularly well suited for scaling-up as it is difficult to achieve the same homogeneous mixing and the same relative mass transport in large reactors as obtained in smaller laboratory or pilot reactors. Besides, the strong agitation implies a significant heating-up of the fermentation fluid.

In order to avoid the mechanical agitation different types of air-lift reactors have been developed. Most of these air-lift reactors are so called loop-reactors of which two parts - an upstream part and a downstream part - form part. One or more gases are supplied at the bottom of the reactor into the upstream part through an arrangement that gives small gas bubbles (e. g. a sintered ceramic disk or a row of small nozzles). The bubbles mix with the fluid whereby the overall density is decreased and the gas-fluid mixture is being displaced by new fluid from the downstream part. The gas-fluid mixture moves up through the upstream part of the reactor and at the top of the reactor the mixture gives off its gas bubbles and after this the fluid moves down through the downstream part. Air-lift reactors are traditionally high slender reactors in order to obtain a long time of stay of the gas bubbles in the fluid. This means that when supplying the gas it has to be introduced under a high pressure to overcome the hydrostatic pressure found at the bottom of the reactor. If the gas is air this involves the use of one or more compressors. Besides, air-lift reactors have a relatively bad utilization of the gas introduced by injection as typically merely 20-40% of the gas is being utilized.

An example of a method carried out using a loop-type reactor as well as a loop-type reactor for executing this method are disclosed in the published EP Patent Application no. 0 185 407.

This known method is directed to the preparation by fermentation of polysaccharides from water, glucose and nutrient salts with air, however, it may also be used for the preparation of yeast or gluconic acid, oxidation of ethen and preparation of single cell protein (SCP) making use of paraffin dispersed in water.

In the prior art method air is supplied into the apparatus via a tube fitted to the lower part of the upstream part, as a result of which the air must be supplied at a pressure of app. 4 bar in order to overcome the pressure in the fermentation fluid, since the known apparatus for use in the implementation of the method will typically be app. 40 metres high in a full scale apparatus.

In addition, the time of stay of the air fed into the apparatus will comprise the time of rising through the upstream part only, since the air will essentially be separated off from the fermentation fluid at the top part of the apparatus.

In the prior art method fermentation fluid is circulated partly by means of the introduction of air at the bottom of the upstream part (like in traditional air-lift reactors) partly by means of a pump provided in the U-part of the apparatus and in the direction of flow before air is fed into the fermentation fluid. At this site the fluid is virtually free of air, as a result of which it is possible to use a gear pump as a circulation pump.

Thus, the method and the apparatus according to EP Patent Application no. 0 185 407 is very energy demanding, since the used air must be condensed into the high pressure indicated, the time of stay of the air in the fermentation fluid is limited, and the pump does not in itself contribute to an intimate mixing between the air and the fermentation fluid.

Besides there is no indication in the above cited application as to how the separation of air and fermentation fluid is carried out by using the known method.

It is therefore an object of the present invention to indicate a method of performing a fermentation, wherein a pressureless or almost pressureless introduction of one or more gases is carried out, a long time of stay is provided, thereby achieving a high degree of utilization of the gas or the gases introduced, a circulation of the fluid is carried out at a low power consumption, a good separation of the gas respectively the gases and the fluid is achieved in the top part of the apparatus used for the implementation of the method, an apparatus of simple design is used, and to provide an apparatus for use in the implementation of this method.

This object is achieved by a method of the initially described kind, which method according to the invention is characteristic in that the gas or the gases are introduced into the fermentation fluid in the downstream part, especially at the top of the downstream part, in that the fermentation fluid is circulated by means of a pump of propeller type placed in the lower end of the upstream part, and in that the fermentation fluid from the upper part of the upstream part is transferred to the top part of the downstream part asymmetrically in relation to the longitudinal axis of the downstream part.

The direction of lift of the gas bubbles is opposite the direction of the fluid circulation in the downstream part, as a consequence of which the resulting velocity of the gas bubbles down through the downstream part will be lower than that of the fluid. In the upstream part, the resulting bubble velocity is equal to the sum of the fluid velocity and the lift velocity of the bubbles. A longer period of contact between the bubbles and the fluid is therefore obtained in the downstream part than in the upstream part. This counter-flow principle thus only works when a pump is applied for circulation of the fluid.

In the implementation of the method according to the invention a very long time of contact is achieved between the gas phase and the fluid phase in that the blown in gas or gases is/are in contact with the fluid phase in the downstream part as well as in the upstream part. This means a substantially higher degree of utilization of the gas or the gases injected as compared to the degree of utilization known from the implementation of the prior art fermentation method, which is carried out making use of a loop-reactor based on the traditional and thus known air-lift principle.

The method according to the present invention for performing a fermentation is carried out mostly continuously but may be carried out discontinuously or batchwise, if desired.

It is advantageous in connection with the method according to the invention that the gas or the gases pass at least one mixer, preferably at least a static mixer, immediately after the introduction.

The amount of gas which can be dispersed or mixed into the fluid depends upon the hydrostatic pressure. In high apparatuses it may therefore be advantageous to have several places for adding gas or gases along the downstream part. The only requirement of each inlet is that a mixing element, preferably a static mixer, has been provided for dispersing of the gas in the fluid immediately after the introduction.

In the implementation of the method according to the invention, gas introduction wil take place at least at the upper end of the downstream part. By introducing the gas or the gases here, an almost pressureless introduction is achieved, since the gas or the gases must overcome a hydrostatic pressure of not more than a few metres. The gas or the gases may be introduced by means of a special gas distributor, which will provide distribution across the downstream part. Fine distribution of the gas or the gases in the fermentation fluid takes place by means of mixers such as static mixers placed immediately below the gas inlet. The fluid flow in the downstream part must be adequately high to the effect that all of the injected gas is carried along through the static mixers. Fine distribution of the gas takes place here to the effect that a large amount of small gas bubbles are dispersed or mixed evenly into the fermentation fluid. The bubbles are carried with the fluid down through the downstream part and further through the U-bend part to the upstream part. One or more static mixers may be provided in the downstream part to the effect that the gas bubbles are redistributed several times in the fermentation fluid.

When executing the method the gas bubbles in the fermentation fluid have a tendency to undergo a fusing-together into bigger units (coalescence). This tendency contributes to render air-lift reactors and hence loop-reactors of the prior art ineffective as the gas bubbles become increasingly bigger through the upstream part partly as a consequence of fusing-together and partly due to a reduced hydrostatic pressure. When carrying out the method according to the invention this tendency in the upstream part is counteracted by placing mixing devices, preferably static mixers, at suitable intervals depending on the rheological properties of the fluid used. In the downstream part the tendency to increased bubble size is counteracted by the steadily increased hydrostatic pressure. To the extent that this effect cannot compensate for the coalescence of the gas bubbles, care is taken for a redistribution of the gas bubbles by means of mixing devices, preferably static mixers.

A propeller pump is advantageously applied when executing the method of the invention, the propeller blades of which pump being constructed for pumping a mixture of fluid and gas.

The pump is preferably placed in the passage between the U-bend and the upstream parts, since it will here be able to serve the purpose of providing redispersion of the gas phase in the fermentation fluid.

Furthermore, it is advantageous in connection with the method to remove the fermentation products from a fermentation fluid virtually free of gas bubbles which is particularly found at the bottom of the U-bend.

Together with a drain pipe and the pump the semi-circular bend form an integral unit. Due to the centrifugal force and the lift of the gas bubbles, the gas which has not been dissolved and consumed in the downstream part will be separated from the fluid at the entry to the U-bend so that the gas bubbles will rest in the uppermost part of the bend, and the fluid at the bottom or radially outermost in the bend (where the drain pipe is found) is almost free of gas bubbles. By continuous draining of the fermentation fluid the need for conducting the fermentation fluid through an air separator is hereby eliminated.

By the method according to the invention the upstream part via a bend is made to enter tangentially on the side of a cylinder having a diameter being larger than the diameter of the downstream part, which cylinder is connected to the downstream part by means of a taper piece. This serves to provide a good separation of the fermentation fluid and gas bubbles as centrifugal forces are acting in the bend as well as in the cylinder on top of the downstream part. The method according to the invention thereby solves one of the great problems associated with air-lift reactors in a very simple manner, viz. separation of gas and fluid phase.

According to the invention, the initially described apparatus is characteristic in that the devices for introduction of the gas or the gases are placed in the downstream part, especially at the top of the downstream part, in that the pump is of propeller type and is placed in the lower end of the upstream part, and in that the upper end of the upstream part has been introduced horizontally and tangentially into the lower end of the cylinder of the top part via a bend.

The apparatus ensures a long period of stay for the gas bubbles in the fermentation fluid, and it is energy saving compared to the known apparatuses since only a low pressure is required in order to introduce gas into the fermentation fluid. Moreover, a good separation between the fermentation fluid and the surplus gas consumed is obtained by the mentioned embodiment of the top part of the apparatus. Also, the fermentation products can be removed in a reliable (bubble free) manner from the U-bend.

The dependent claims relate to expedient embodiments of the apparatus.

An example of a preferred embodiment of an apparatus to be used for carrying out the method according to the invention will be explained below with reference to the drawing.

In the drawing
- fig. 1: shows an embodiment of the apparatus of the invention seen from the side and for the part of major parts of the apparatus also in section and
- fig. 2: shows the apparatus in fig. 1 seen from above.

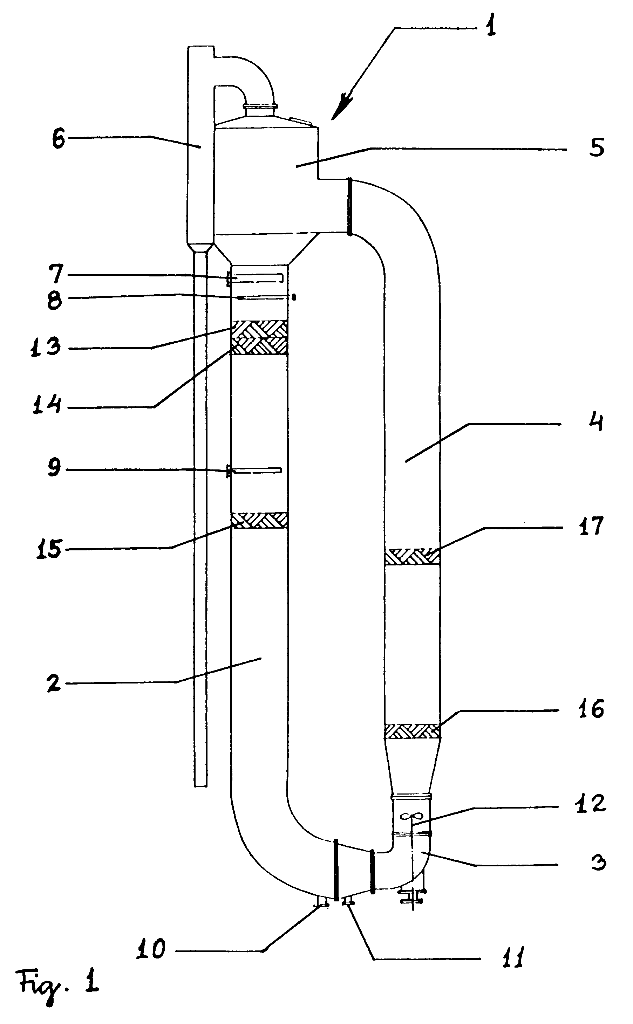

In fig. 1 the numeral 1 designates an embodiment of the apparatus of the invention. 2 designates its downstream part, 3 its U-bend part and 4 its upstream part. The downstream part 2, the U-bend part 3 and the upstream part 4 together make up the U-part of the apparatus. 5 designates its top part while 6 is a venting duct. 7 designates a first device for introducing air, 8 a second device for introducing a gas, and 9 a device for introducing oxygen, it hereby being observed that air as it is known is a mixture of gases and that oxygen is an example of a gas. 11 designates a pipe for supplying water, nutrition salts and micro-organisms, such as bacteria, and 10 a pipe for removing fermentation fluid (including the fermentation products, such as bacteria) present during or after a fermentation. 12 designates an in-line pump. 13, 14, 15, 16, and 17 each designates a static mixer.

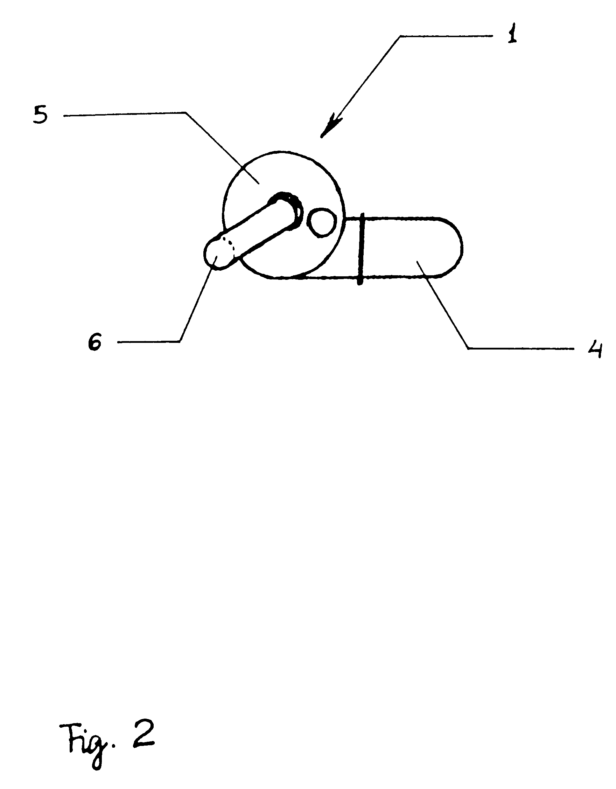

In fig. 2 the numerals 4, 5, and 6 designate the same parts of the apparatus as in fig. 1. In fig. 2 there is i. a. illustrated the below mentioned tangential entering of the upstream part 4 via a bend, this entering being mentioned in connection with the mention of the top part 5.

In order to give an impression of the dimensions an apparatus 1 of the invention may have by way of example it may be mentioned that the total height may be 40 metres and that its width may be 6.6 metres in that by width is understood the distance between the walls of the downstream part 2 and the walls of the upstream part 4 being longest apart. The inside diameter, d, of the downstream part 2 and of the upstream part 4 may in both cases be 1.65 metres respectively. The radius of the bend at the end of the downstream part 2 and the upstream part 4 may be 1.5 x d respectively.

In apparatus 1 of the invention, the gas phase (in the form of small bubbles) is in contact with the fluid phase both in the downstream part 2 and in the upstream part 4. This gives a much higher degree of utilization of the injected gas or gases than known from loop-reactors based on the air-lift principle.

The top part 5 of the apparatus 1 of the invention has been worked out in such a way that the upstream part 4 via a bend has been made to enter on the side of a cylinder, i. e. tangentially on a cylinder, having a diameter that is greater than the diameter of the downstream part 2 and being connected to the downstream part by means of a taper piece. This special design contributes to giving a good separation of fluid and gas bubbles by using centrifugal forces.

The gases are introduced in the upper end of the downstream part by means of particular gas distributors taking care of the distribution across the downstream part. The comminution of the gases and the subsequent distribution of the finely divided bubbles in the fluid takes place by means of static mixers, 13, 14 and 15 respectively, placed immediately below the gas inlets.

## Claims

1. A method for performing a fermentation in which method an introduction and a subsequent mixing of one or more gases is carried out in a fermentation fluid, which is found in an apparatus of the loop-reactor type, which apparatus has a top part and a U-part consisting of a downstream part, a U-bend part and an upstream part, the fermentation fluid being circulated in the apparatus by means of a pump placed in the U-part, **characterized** in that the gas or the gases are introduced into the fermentation fluid in the downstream part (2), especially at the top of the downstream part, in that the fermentation fluid is circulated by means of a pump of propeller type placed in the lower end of the upstream part (4), and in that the fermentation fluid from the upper part of the upstream part (4) is transferred to the top part (5) of the downstream part (2) asymmetrically in relation to the longitudinal axis of the downstream part (2).

2. A method according to claim 1, **characterized** in that the gas or the gases are mixed into the fermentation fluid by means of one or more mixers placed in the downstream part (2) and the upstream part (4), especially at sites immediately after introduction of gases (7, 8, 9), said mixers being preferably static mixers (13, 14, 15 respectively 16, 17).

3. A method according to claims 1 or 2, **characterized** in that the fermentation product such as micro-organism cell mass is being removed in the lower end of the downstream part (2).

4. An apparatus for use in the method according to claim 1, which apparatus is of the loop reactor type and has a top part (5) and a U-part consisting of a substantially vertical downstream part (2), a U-bend part (3) and a substantially vertical upstream part (4), a pump (12) placed in the U-part, which pump is adapted to circulate a fermentation fluid in the apparatus, and devices for introduction of one or more gases into the fermentation fluid, whereby the top part (5) is made up of a cylinder having a substantially larger diameter than the downstream part (2) and being connected hereto via a taper part, **characterized** in that the devices (7, 8, 9) for introduction of the gas or the gases are placed in the downstream part (2), especially at the top of the downstream part, in that the pump (12) is of propeller type and is placed in the lower end of the upstream part (4), and in that the upper end of the upstream part (4) has been introduced horizontally and tangentially into the lower end of the cylinder of the top part (5) via a bend.

5. An apparatus according to claim 5, **characterized** in that the apparatus has one or more mixers placed in the downstream part (2) and the upstream part (4), especially at sites immediately after introduction of gases (7, 8, 9), said mixers being preferably static mixers (13, 14, 15 respectively 16, 17).

6. An apparatus according to claims 4 or 5, **characterized** in that the apparatus has devices (10) in the lower end of the downstream part (2), which are adapted for removal of fermentation products, in particular micro-organism cell mass resulting from the fermentation.

## Patentansprüche

1. Verfahren zum Durchführen einer Fermentation, bei dem das Einleiten und das anschließende Vermischen eines Gases oder mehrerer in einer Fermentationsflüssigkeit ausgeführt werden, die sich in einer Vorrichtung vom Rohrschlaufenreaktor-Typ befindet, die einen oberen Teil und einen U-förmigen Teil, der aus einem Abwärtsströmungsteil, einem U-förmig gebogenen Teil und einem Aufwärtsströmungsteil besteht, aufweist, wobei die Fermentationsflüssigkeit in der Vorrichtung durch eine Pumpe umgewälzt wird, die im U-förmigen Teil angeordnet ist, **dadurch gekennzeichnet,** daß das Gas oder die Gase im Abwärtsströmungsteil (2), insbesondere im oberen Teil des Abwärsströmungsteils in die Fermentationsflüssigkeit eingeleitet werden, daß die Fermentationsflüssigkeit durch eine Rotationspumpe umgewälzt wird, die im unteren Ende des Aufwärtsströmungsteils (4) angeordnet ist, und daß die Fermentationsflüssigkeit vom oberen Teil im Aufwärtsströmungsteil (4) asymmetrisch in bezug auf die Längsachse des Abwärtsströmungsteils (2) zum oberen Teil (5) des Abwärtsströmungsteils (2) transportiert wird.

2. Verfahren nach Anspruch 1, **dadurch gekennzeichnet,** daß das Gas oder die Gase durch einen oder mehrere Mischer in die Fermentationsflüssigkeit eingemischt werden, die im Abwärtsströmungsteil (2) und im Aufwärtsströmungsteil (4) insbesondere an Orten direkt nach der Einleitung von Gasen (7, 8, 9) angeordnet sind, wobei die Mischer vorzugsweise statische Mischer (13, 14, 15 bzw. 16, 17) sind.

3. Verfahren nach einem der Ansprüche 1 oder 2, **dadurch gekennzeichnet,** daß das Fermentationserzeugnis wie eine Mikroorganismus-Zellenmasse am unteren Ende des Abwärtsströmungsteils (2) entnommen wird.

4. Vorrichtung zur Verwendung beim Verfahren gemäß Anspruch 1, die vom Rohrschlaufenreaktor-Typ ist und über folgendes verfügt: einen oberen Teil (5) und einen U-förmigen Teil, der aus einem im wesentlichen vertikalen Abwärtsströmungsteil (2), einem U-förmig gebogenen Teil (3) und einem im wesentlichen vertikalen Aufwärtsströmungsteil (4) besteht, eine Pumpe (12), die im U-förmigen Teil angeordnet ist und so ausgebildet ist, daß sie eine Fermentationsflüssigkeit in der Vorrichtung umwälzt, und Vorrichtungen zum Einleiten eines Gases oder mehrerer in die Fermentationsflüssigkeit, wobei der obere Teil (5) aus einem Zylinder besteht, der einen wesentlich größeren Durchmesser als der Abwärtsströmungsteil (2) aufweist, der über einen sich verjüngenden Teil damit verbunden ist; **dadurch gekennzeichnet,** daß die Vorrichtungen (7, 8, 9) zum Einleiten des Gases oder der Gase im Abwärtsströmungsteil (2), insbesondere oben am Abwärtsströmungsteil, angeordnet sind, daß die Pumpe (12) vom Radialtyp ist und im unteren Ende des Aufwärtsströmungsteils (4) angeordnet ist, und daß das obere Ende des Aufwärtsstromungsteils (4) horizontal und tangential über einen gebogenen Teil in das untere Ende des Zylinders des oberen Teils (5) eingeführt ist.

5. Vorrichtung nach Anspruch 5, **dadurch gekennzeichnet,** daß sie einen Mischer oder mehrere aufweist, die im Abwärtsströmungsteil (2) und im Aufwärtsströmungsteil (4) insbesondere an Orten direkt nach der Einleitung der Gase (7, 8, 9) angeordnet sind, wobei die Mischer vorzugsweise statische Mischer (13, 14, 15 bzw. 16, 17) sind.

6. Vorrichtung nach einem der Ansprüche 4 oder 5, **dadurch gekennzeichnet,** daß sie Vorrichtungen (10) im unteren Ende des Abwärtsströmungsteils (2) aufweist, die zur Entnahme von Fermentationserzeugnissen ausgebildet sind, insbesondere einer Mikroorganismus-Zellenmasse, die von der Fermentation herrührt.

## Revendications

1. Procédé pour réaliser une fermentation, dans lequel il y a introduction puis mélange d'un ou plusieurs gaz dans un fluide de fermentation qui se trouve dans un appareil du type réacteur en boucle, lequel appareil a une partie de tête et une partie en U consistant en une partie d'écoulement descendant, une partie coudée en U et une partie d'écoulement ascendant, le fluide de fermentation étant mis en circulation dans l'appareil au moyen d'une pompe placée dans la partie en U, caractérisé en ce que le ou les gaz sont introduits dans le fluide de fermentation dans la partie d'écoulement descendant (2), particulièrement au sommet de la partie d'écoulement descendant, en ce que le fluide de fermentation est mis en circulation au moyen d'une pompe du type à pales placée dans l'extrémité inférieure de la partie d'écoulement ascendant (4), et en ce qu'à partir de la partie supérieure de la partie d'écoulement ascendant (4) le fluide de fermentation est transféré à la partie de tête (5) de la partie d'écoulement descendant (2) asymétriquement par rapport à l'axe longitudinal de la partie d'écoulement descendant (2).

2. Procédé selon la revendication 1, caractérisé en ce que le ou les gaz sont mélangés dans le fluide de fermentation au moyen d'un ou plusieurs mélangeurs placés dans la partie d'écoulement descendant (2) et la partie d'écoulement ascendant (4), particulièrement en des emplacements situés immédiatement après l'introduction des gaz (7, 8, 9), ces mélangeurs étant de préférence des mélangeurs statiques (13, 14, 15 respectivement 16, 17).

3. Procédé selon les revendications 1 ou 2, caractérisé en ce que le produit de fermentation tel qu'un amas formé à partir de cellules de micro-organismes est prélevé à l'extrémité inférieure de la partie d'écoulement descendant (2).

4. Appareil destiné à être utilisé dans le procédé selon la revendication 1, lequel appareil est du type réacteur en boucle et possède une partie de tête (5) et une partie en U consistant en une partie d'écoulement descendant sensiblement verticale (2), une partie coudée en U (3) et une partie d'écoulement ascendant sensiblement verticale (4), une pompe (12) placée dans la partie en U et adaptée à faire circuler un fluide de fermentation dans l'appareil, et des dispositifs pour introduire un ou plusieurs gaz dans le fluide de fermentation, de sorte que la partie de tête (5) est constituée d'un cylindre ayant un diamètre sensiblement plus grand que la partie d'écoulement descendant (2) en étant reliée à celle-ci par l'intermédiaire d'une partie allant en se rétrécissant, caractérisé en ce que les dispositifs (7, 8, 9) pour introduire le ou les gaz sont placés dans la partie d'écoulement descendant (2), particulièrement au sommet de la partie d'écoulement descendant, en ce que la pompe (12) est du type à pales et se trouve placée dans l'extémité inférieure de la partie d'écoulement ascendant (4), et en ce que l'extrémité supérieure de la partie d'écoulement ascendant (4) a été introduite horizontalement et tangentiellement dans l'extrémité inférieure du cylindre de la partie de tête (5) par l'intermédiaire d'un coude.

5. Appareil selon la revendication 5, caractérisé en ce que l'appareil possède un ou plusieurs mélangeurs placés dans la partie d'écoulement descendant (2) et la partie d'écoulement ascendant (4), particulièrement en des emplacements situés immédiatement après l'introduction des gaz (7, 8, 9), ces mélangeurs étant de préférence des mélangeurs statiques (13, 14, 15 respectivement 16, 17).

6. Appareil selon les revendications 4 ou 5, caractérisé en ce que l'appareil possède dans l'extrémité inférieure de la partie d'écoulement descendant (2) des dispositifs (10) adaptés au prélèvement de produits de fermentation, en particulier un amas formé à partir de cellules de micro-organismes, résultant de cette fermentation.
